# EUROPEAN PATENT SPECIFICATION

(11) **EP 1 642 981 B1**
(45) Date of publication and mention of the grant of the patent: **15.06.2011**
(21) Application number: 04731832.4
(22) Date of filing: 09.05.2004
(51) Int. Cl.: C12N 15/86, C12N 15/12, A61K 48/00, A61P 17/00

(54) **RECOMBINANT GENE MEDICINE OF ADENOVIRUS VECTOR AND GENE p53 FOR TREATING PROLIFERATIVE DISEASES**
REKOMBINANTE GENMEDIZIN AUS ADENOVIRUSVEKTOR UND GEN P53 ZUR BEHANDLUNG PROLIFERATIVER ERKRANKUNGEN
MEDECINE PAR GENE DE RECOMBINAISON DE VECTEUR ADENOVIRUS ET GENE p53 DESTINES A TRAITER DES MALADIES PROLIFERATIVES

(30) Priority: 10.05.2003 CN 03125129
(43) Date of publication of application: 05.04.2006
(73) Proprietor: Peng, Zhaohui,, Shenzhen, Guangdong 518057 (CN); Zhang, Xiaozhi, Shenzhen, Guangdong 518057 (CN)
(72) Inventor: Peng, Zhaohui,, Shenzhen, Guangdong 518057 (CN); Zhang, Xiaozhi, Shenzhen, Guangdong 518057 (CN)
(74) Representative: Ebner von Eschenbach, Jennifer
(86) International application number: PCT/CN2004/000458
(87) International publication number: WO 2004/104204

(56) References cited:
- WO-A-98/40508
- CN-A- 1 401 778
- US-B1- 6 476 206
- SASAKI R ET AL: "Additional gene therapy with Ad5CMV-p53 enhanced the efficacy of radiotherapy in human prostate cancer cells." INTERNATIONAL JOURNAL OF RADIATION ONCOLOGY, BIOLOGY, PHYSICS. 1 DEC 2001, vol. 51, no. 5, 1 December 2001 (2001-12-01), pages 1336-1345, XP002389766 ISSN: 0360-3016
- SHIRAKAWA T ET AL: "p53 adenoviral vector (Ad-CMV-p53) induced prostatic growth inhibition of primary cultures of human prostate and an experimental rat model." THE JOURNAL OF GENE MEDICINE. 2000 NOV-DEC, vol. 2, no. 6, November 2000 (2000-11), pages 426-432, XP002389767 ISSN: 1099-498X
- HE ET AL: "A simplified system for generating recombinant adenoviruses" PROCEEDINGS OF THE NATIONAL ACADEMY OF SCIENCES OF USA, NATIONAL ACADEMY OF SCIENCE, WASHINGTON, DC, US, vol. 95, no. 5, 3 March 1998 (1998-03-03), pages 2509-2514, XP002109113 ISSN: 0027-8424
- TEOFOLI P. ET AL.: 'Expression of Bcl-2, p53, c-jun and c-fos protooncogenes in Keloids and hypertrophic scars' JOURNAL OF DERMATOLOGICAL SCIENCE vol. 22, no. 1, December 1999, pages 31 - 37, XP002998360

### SEQUENCE

<110> PENG, zhaohui etc.
<120> Recombinant gene medicine of Adenovirus vector and P53 gene for Treating Proliferative Diseases
<130> CPS41234
<140> PCT/CN2004/000458
<141> 2004-05-09
<150> CN03125129.3
<151> 2003-05-10
<160> 1
<170> PatentIn version 3.1
<210> 1
<211> 2848
<212> DNA
<213> Artifical sequence
<400> 1

atgtttaccg ccacactcgc agggtctgca cctggtgcgg gtctcatcgt acctcagcac 60
cttccagatc tctgacatgc gatgtcgact cgactgcttc gcgatgtacg ggccagatat 120
acgcgtatct gaggggacta gggtgtgttt aggcgaaaag cggggcttcg gttgtacgcg 180
gttaggagtc ccctcaggat atagtagttt cgcttttgca tagggagggg gaaatgtagt 240
cttatgcaat actcttgtag tcttgcaaca tggtaacgat gagttagcaa catgccttac 300
aaggagagaa aaagcaccgt gcatgccgat tggtggaagt aaggtggtac gatcgtgcct 360
tattaggaag gcaacagacg ggtctgacat ggattggacg aaccactgaa ttccgcattg 420
cagagatatt gtatttaagt gcctagctcg atacaataaa cgccatttga ccattcacca 480
cattggtgtg cacctccaag cttggtaccg agctcggatc ccgctagagc caccgtccag 540
ggagcaggta gctgctgggc tccggggaca ctttgcgttc gggctgggag cgtctttcca 600
cgacggtgac acgcttccct ggattggcag ccagactgct ttccgggtca ctgccatgga 660
ggagccgcag tcagatccta gcgtcgagcc ccctctgagt caggaaacat tttcagacct 720
atggaaacta cttcctgaaa acaacgttct gtcccccttg ccgtcccaag caatggatga 780
tttgatgctg tccccggacg atattgaaca atggttcact gaagacccag gtccagatga 840
agctcccaga atgccagagg ctgctccccc cgtggcccct gcaccagcag ctcctacacc 900
ggcggcccct gcaccagccc cctcctggcc cctgtcatct tctgtccctt cccagaaaac 960
ctaccagggc agctacggtt tccgtctggg cttcttgcat tctgggacag ccaagtctgt 1020

gacttgcacg tactcccctg ccctcaacaa gatgttttgc caactggcca agacctgccc 1080
tgtgcagctg tgggttgatt ccacaccccc gcccggcacc cgcgtccgcg ccatggccat 1140
ctacaagcag tcacagcaca tgacggaggt tgtgaggcgc tgcccccacc atgagcgctg 1200
ctcagatagc gatggtctgg cccctcctca gcatcttatc cgagtggaag gaaatttgcg 1260
tgtggagtat ttggatgaca gaaacacttt tcgacatagt gtggtggtgc cctatgagcc 1320
gcctgaggtt ggctctgact gtaccaccat ccactacaac tacatgtgta acagttcctg 1380
catgggcggc atgaaccgga ggcccatcct caccatcatc acactggaag actccagtgg 1440
taatctactg ggacggaaca gctttgaggt gcgtgtttgt gcctgtcctg ggagagaccg 1500
gcgcacagag gaagagaatc tccgcaagaa aggggagcct caccacgagc tgcccccagg 1560
gagcactaag cgagcactgc ccaacaacac cagctcctct ccccagccaa agaagaaacc 1620
actggatgga gaatatttca cccttcagat ccgtgggcgt gagcgcttcg agatgttccg 1680
agagctgaat gaggccttgg aactcaagga tgcccaggct gggaaggagc caggggggag 1740
cagggctcac tccagccacc tgaagtccaa aaagggtcag tctacctccc gccataaaaa 1800
actcatgttc aagacagaag ggcctgactc agactgacat tctccacttc ttgttcccca 1860
ctgacagcct cccaccccca tctctccctc ccctgccatt ttgggttttg ggtctttgaa 1920
cccttgcttg caataggtgt gcgtcagaag cacccaggac ttccatttgc tttgtcccgg 1980
ggctccactg aacaagttgg cctgcactgg tgttttgttg tggggaggag gatggggagt 2040
aggacatacc agcttagatt ttaaggtttt tactgtgagg gatgtttggg agatgtaaga 2100
aatgttcttg cagttaaggg ttagtttaca atcagccaca ttctaggtag gggcccactt 2160
caccgtacta accagggaag ctgtccctca ctgttgaatt ttctctaact tcaaggccca 2220
tatctgtgaa atgctggatt tgccctacct cggaatgctg gcatttgcac ctacctcaca 2280
gagtgcattg tgagggttaa tgaaataatg tacatctggc cttgaaacca cttttattac 2340
atggggtcta gcgggatcca ctagtaacgc cgccagtgtg ctggaattct gcagatatcc 2400
atcacactgg cggccgctcg agcatgcatc tagagctcgc tgatcagcct cgactgtgcc 2460
ttctagttgc cagccatctg ttgtttgccc ctcccccgtg ccttccttga ccctggaagg 2520
tgccactccc actgtccttt cctaataaaa tgaggaaatt gcatcgcatt gtctgagtag 2580
gtgtcattct attctggggg gtggggtggg gcaggacagc aagggggagg attgggaaga 2640
caatagcagg catgctgggg atgcggtggg ctctatggct tctgaggcgg aaagaaccag 2700
ctggggctcg agggggatcc ccacgctaga gctgactata ataataaaac gccaactttg 2760
acccggaacg cggaaaacac ctgagaaaaa cacctgggcg agtctccacg taaacggtca 2820
aagtccccgc ggccctagac aaatatta 2846

## Description

### Field of the Invention

The present invention relates to genetic engineering technology, especially to the genetic recombinant medicine that is made of the recombinant sequences of human tumor suppressor gene p53 and adenovirus vector for treatment of proliferative diseases.

### Background of the Invention

Proliferative disease is a genic disease featured by cell hyperplasia and/or abnormal expression of metabolite products. It is a benign hyperplasia, which could cause dysfunctions in all human tissues and organs such as skin, marrow and mammary gland. The scar is the inevitable outcome of the human wound recovering process. Any kind of wound recovering will result in scars. There are two kinds of scars including normal scar and pathological scar. Pathological scar includes hyperplastic scar and cheloid, which both cause neoplastic proliferation and dysfunction. It is one of the four most difficult problems in medical science. Cheloid is a skin hyperplastic disease. It is over hyperplasia caused by voluntarily or involuntarily induced abnormal collagen accumulation that usually occurs in skin injury. Its clinical symptoms include over development, over the original border of the injury, invasion of the neighbor tissues, non-recession, and reoccurrence after surgery. It has been proved by many experiments that p53 gene mutation occurs in cheloid formation. However, there is still no effective and specific treatment for cheloid in nowadays. It is also one of the most important problems in orthopaedic surgery.

The vectors used in gene therapy solely have not curative potential =. On the other hand, the genes which could be used to treat the diseases only have potential treating capability, since it is very difficult for them to directly enter and then express in the target cells. In order for the potential therapeutic genes to take effect, the vector should first be recombined with the gene, then carry the gene into the target cells by transfection, and finally the gene could enter the cells and be expressed. Therefore, the key of gene therapy is to construct the recombinant DNA for the therapeutic gene and the gene vector.

The common way to recombine the target gene expression cassette with its vector is to carry out homologous recombination in eukaryotic cells, which is a very complicated and tedious process. However, using prokaryotic cells for homologous recombination and to construct recombinant vectors could solve the above problems.

A bottleneck in gene therapy lies in the lack of specific, targeting and efficient gene vectors. At present, there are two kinds of vectors used in gene therapy research including viral vectors and non-viral vectors. The common viral vectors include adenovirus vectors, adeno-associated virus vectors and retrovirus vectors. Adenovirus vector is the most common. Its advantages include a high transfection rate, its relative safety and ease of operability, the ability to carry large gene fragments and the ability to prepare high titer viral particles, suitable for production, and the ability to infect cells not only in division phase but also in non-division phase. However, its disadvantages include a lack of target-specific infection and production of immunogenicity. Therefore, it is necessary to improve adenovirus vector for gene therapy. Research indicates that a gene carried by adenovirus vector could be expressed in a longer period time and the antigenicity of the vector could be decreased if the gene Is carried at E1 or E3 missing area. The retrovirus vector could carry a foreign gene and integrate into the target cell's genome, thus realizing the stable and lasting gene expressions. However, the retrovirus has the following disadvantages: low reproduction titer *in vitro*, low transfection efficiency, infecting only the cells in division phase, and random recombination with chromosomes bringing potentially carcinogenic activity. Other viral or non-viral vectors used in gene transformations all have different advantages and disadvantages.

### Summary of Invention

The object of this invention is to recombine the potentially therapeutic genes with their vectors, thus providing a recombinant DNA of adenoviral vector and p53 gene for treatment of hyperplastic scars. This recombinant product will then induce the hyperplastic cells express normal P53 proteins. In this way the proliferation of the abnormal cells could be effectively repressed and could be used to treat hyperplastic scars such as cheloid.

The object of this invention is also to provide a method for producing this recombination and its preparation so that it could be put in practices.

This invention provides recombinant DNA of adneoviral vector and p53 gene. This recombination is constructed by combining adenoviral vector and human tumor suppressor gene expression cassette, which has the following sequence: the right end of adenovirus

ATGTTTACCGCCACACTCGCAGGGTCTGCACCTGGTGCGGGTCTCATCGTAC
CTCAGCACCTTCCAGATC70TCTGACATGCGATGTCGACTCGACTGCTTCGCG
ATGTACGGGCCAGATATACGCGTATCTGAGGGGACTAGGGTGTGTTTAGGCG
AAAAGCGGGGCTTCGGTTGTACGCGGTTAGGAGTCCCCTCAGGATATAGTAG
TTTCGCTTTTGCATAGGGAGGGGGAAATGTAGTCTTATGCAATACTCTTGTAG

TCTTGCAACATGGTAACGATGAGTTAGCAACATGCCTTACAAGGAGAGAAAAA
GCACCGTGCATGCCGATTGGTGGAAGTAAGGTGGTACGATCGTGCCTTATTA
GGAAGGCAACAGACGGGTCTGACATGGATTGGACGAACCACTGAATTCCGCA
TTGCAGAGATATTGTATTTAAGTGCCTAGCTCGATACAATAAACGCCATTTGAC
CATTCACCACATTGGTGTGCACCTCCAAGCTTGGTACCGAGCTCGGATCCCG5
23CTAGAGCCACCGTCCAGGGAGCAGGTAGCTGCTGGGCTCCGGGGACACTT
TGCGTTCGGGCTGGGAGCGTCTTTCCACGACGGTGACACGCTTCCCTGGATT
GGCAGCCAGACTGCTTTCCGGGTCACTGCC655ATGGAGGAGCCGCAGTCAGA
TCCTAGCGTCGAGCCCCCTCTGAGTCAGGAAACATTTTCAGACCTATGGAAAC
TACTTCCTGAAAACAACGTTCTGTCCCCCTTGCCGTCCCAAGCAATGGATGAT
TTGATGCTGTCCCCGGACGATATTGAACAATGGTTCACTGAAGACCCAGGTC
CAGATGAAGCTCCCAGAATGCCAGAGGCTGCTCCCCCCGTGGCCCCTGCAC
CAGCAGCTCCTACACCGGCGGCCCCTGCACCAGCCCCCTCCTGGCCCCTGT
CATCTTCTGTCCCTTCCCAGAAAACCTACCAGGGCAGCTACGGTTTCCGTCTG
GGCTTCTTGCATTCTGGGACAGCCAAGTCTGTGACTTGCACGTACTCCCCTG
CCCTCAACAAGATGTTTTGCCAACTGGCCAAGACCTGCCCTGTGCAGCTGTG
GGTTGATTCCACACCCCCGCCCGGCACCCGCGTCCGCGCCATGGCCATCTA
CAAGCAGTCACAGCACATGACGGAGGTTGTGAGGCGCTGCCCCCACCATGA
GCGCTGCTCAGATAGCGATGGTCTGGCCCCTCCTCAGCATCTTATCCGAGTG
GAAGGAAATTTGCGTGTGGAGTATTTGGATGACAGAAACACTTTTCGACATAG
TGTGGTGGTGCCCTATGAGCCGCCTGAGGTTGGCTCTGACTGTACCACCATC
CACTACAACTACATGTGTAACAGTTCCTGCATGGGCGGCATGAACCGGAGGC
CCATCCTCACCATCATCACACTGGAAGACTCCAGTGGTAATCTACTGGGACG
GAACAGCTTTGAGGTGCGTGTTTGTGCCTGTCCTGGGAGAGACCGGCGCACA
GAGGAAGAGAATCTCCGCAAGAAAGGGGAGCCTCACCACGAGCTGCCCCCA
GGGAGCACTAAGCGAGCACTGCCCAACAACACCAGCTCCTCTCCCCAGCCAA
AGAAGAAACCACTGGATGGAGAATATTTCACCCTTCAGATCCGTGGGCGTGA
GCGCTTCGAGATGTTCCGAGAGCTGAATGAGGCCTTGGAACTCAAGGATGCC
CAGGCTGGGAAGGAGCCAGGGGGGAGCAGGGCTCACTCCAGCCACCTGAA
GTCCAAAAAGGGTCAGTCTACCTCCCGCCATAAAAAACTCATGTTCAAGACAG
AAGGGCCTGACTCAGACTGA1837CATTCTCCACTTCTTGTTCCCCACTGACAGC
CTCCCACCCCCATCTCTCCCTCCCCTGCCATTTTGGGTTTTGGGTCTTTGAAC
CCTTGCTTGCAATAGGTGTGCGTCAGAAGCACCCAGGACTTCCATTTGCTTTG
TCCCGGGGCTCCACTGAACAAGTTGGCCTGCACTGGTGTTTTGTTGTGGGGA
GGAGGATGGGGAGTAGGACATACCAGCTTAGATTTTAAGGTTTTTACTGTGAG
GGATGTTTGGGAGATGTAAGAAATGTTCTTGCAGTTAAGGGTTAGTTTACAAT
CAGCCACATTCTAGGTAGGGGCCACTTCACCGTACTAACCAGGGAAGCTGTC
CCTCACTGTTGAATTTTCTCTAACTTCAAGGCCCATATCTGTGAAATGCTGGAT
TTGCCCTACCTCGGAATGCTGGCATTTGCACCTACCTCACAGAGTGCATTGTG
AGGGTT2297AATGAAATAATGTACATCTGGCCTTGAAACCACCTTTTATTACATG
GGGTCTAGCGGATCCACTAGTAACGCCGCCAGTGTGCTGGAATTCTGCAGA
TATCCATCACACTGGCGGCCGCTCGAGCATGCATCTAGAGCTCGCTGATCAG
CCTCGACTGTGCCTTCTAGTTGCCAGCCATCTGTTGTTTGCCCCTCCCCCGTG

CCTTCCTTGACCCTGGAAGGTGCCACTCCCACTGTCCTTTCCTAATAAAATGA
GGAAATTGCATCGCATTGTCTGAGTAGGTGTCATTCTATTCTGGGGGGTGGG
GTGGGGCAGGACAGCAAGGGGGAGGATTGGGAAGACAATAGCAGGCATGCT
GGGGATGCGGTGGGCTCTATGGCTTCTGAGGCGGAAAGAACCAGCTGGGGC
TCGAGGGGGATCCCCACGCTAGAGCT2733GACTATAATAATAAAACGCCAACT
TTGACCCGGAACGCGGAAAACACCTGAGAAAAACACCTGGGCGAGTCTCCAC
GTAAACGGTCAAAGTCCCCGCGGCCCTAGACAAATATTA 2848- the left end of
adenovirus 5

In which:
1. the right end of adenovirus 5 and the left end of adenovirus 5end are described in adenovirus 5 gene full sequence (Genbank No: NC_001406)
2. 1-70: the right arm of adenovirus (the 70^{th} base locates at adenovirus gene sequence 3328)
3. 71-523□Rous Sarcoma Virus (RSV) LTR (promoter)
4. 524-655: 5' end non-translating region
5. 656-1837: p53 gene coding sequence
6. 1838-2733: 3' end non-translating region (poly Adenosine tail starting at 2298) 2734-2848: the left arm of adenovirus (base at 2734 is positioned at 452 of adenovirus 5 gene sequence).

The expression cassette of this recombination is a specific sequence composed of promoter-p53cDNA-poly Adenosine. Its upstream sequence contains any eukaryotic cell promoter, prokaryotic cell promoter or virus promoter. Its downstream sequence contains any poly adenosine (polyA) of eukaryotic cells.

The recombinant DNA of this invention was obtained as described below. The recombinant virus vector was obtained in prokaryotic cells by homologous recombination. First, the recombinant product pGT-2 was constructed in *E.coli* by homologous recombination of the adenovirus with plasma pGT-1 which included the adenovirus's inverted repeat sequences on both ends. Second, recombinant product pGT-3 was constructed in *E.coli* by homologous recombination of pGT-2 with the artificial sequence "adenovirus right arm/promoter-p53cDNA-poly adenosine/adenovirus left arm". Finally, recombinant p53 adenovirus was obtained by discarding the prokaryotic plasmid sequence using endonuclease *Pac*l.

In fact, this recombination could be obtained from any prokaryotic cell by homologous recombination.

According to the above methods, the long terminal repeats (LTR) on both sides of adenovirus were amplified by PCR and *Pac*l restriction enzymatic sites were introduced respectively. Both LTR fragments were cloned into pUC18 vector and produced pGT-1 recombinant sequence. The constructed pGT-1 vector and adenovirus 5 gene sequence were then co-transfected the *E.coli* (BJ5183, preserved by SiBiono Company, preserve no.: P-e012). The adenovirus 5 gene sequence was then homologously combined with pGT-1. The positive virus clone was then amplified, screened by PCR, and tested using restriction enzymes. Finally, recombination vector pGT-2 which contains adenovirus 5 full gene sequence was obtained.

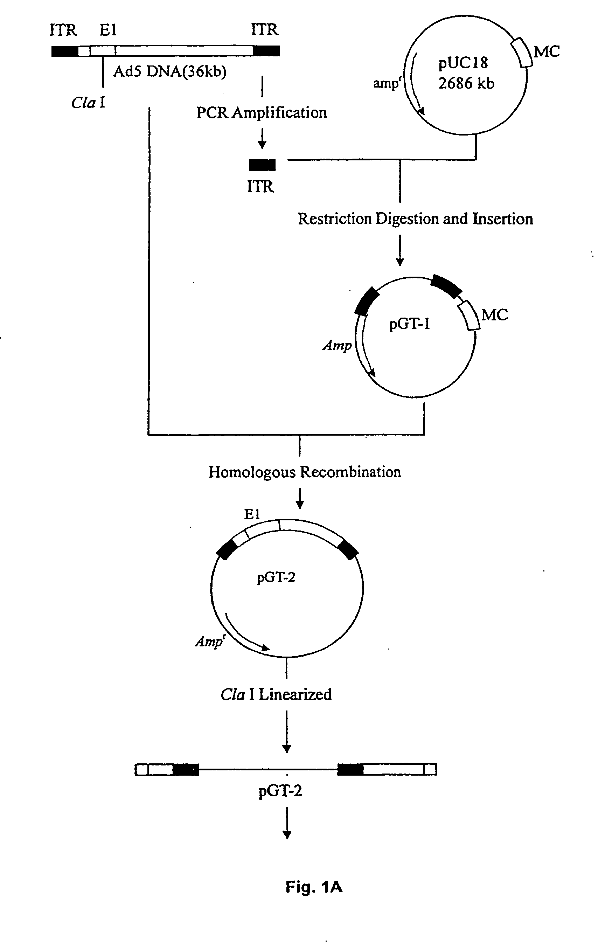

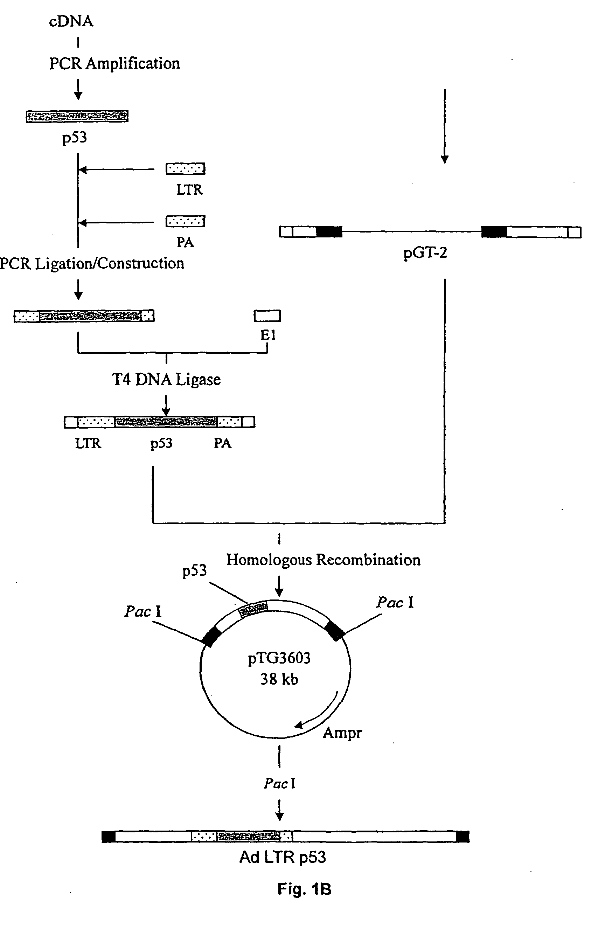

Using 5' ATGGAGGAGCCGCAGTCAGATC and 5' ATATCTGCAGAATTCCAGC AC as a primer, human tumor suppressor p53 gene was amplified by PCR. The full sequence of p53 gene (including the 5' and 3' non-translation sequence) was then cloned into vector pUC19 and tested by DNA sequencing. Next, the RSV (rous sarcoma virus) LTR sequence and the PA sequence of BGH and E1 sequence of adenovirus were amplified by PCR. A linker sequence was attached on one side of each aforesaid sequence and confirmed by DNA sequencing. In the next PCR reaction, LTR and PA sequences were attached to the 5' and 3' ends of p53, respectively. The adenovirus E1 section and its upstream sequence were combined to the outer side of p53, and the p53 gene expression cassette was thus constructed (Figure 1).

The *E.coli* BJ5183 was co-transfected by recombinant vector pGT-2 and p53 gene expression cassette, in which the homologous recombination occurred. The positive clones were then amplified, PCR screened, and tested via restriction enzymes digestion. The resulting recombinant vector was pGT-3, which contained most of the adenovirus 5 sequence (its E1 section and part of the upstream sequence were substituted by p53 expression cassette). The recombinant vector pGT-3 was linearized by Pad and the sequence which originated from pUC18 was discarded. Then the pGT-3 was used to transfect 293 cells (preserved by SiBiono Corp., preservation No.: E-393). The recombinant was packed in cells and produced human tumor suppressor gene p53 cassette containing adenovirus cis-acting sequence and LTR promoter. The resulting recombinant p53 adenovirus vector had a high transfection rate, was easy to operate and was controlled by a single promoter.

This recombinant p53 adenovirus had the following characteristics:

It was constructed with adenovirus vector and p53 gene artificial expression cassette,
1. Structure: It was a living recombinant adenovirus which was different from other chemical synthetic medicines, herbs and genetically engineered medicines. It was highly biologically active, could be directly expressed *in vivo* and was highly effective in clinical application. Adenovirus could carry large gene fragment and had high transfection rate, which could be made as high titer virus particles and had a very broad host range, proved very safe. Its antigenicity was greatly reduced especially after being reconstructed. Thus the target gene was easily stabilized and expressed *in vivo*. In p53 artificial gene expression cassette, the expression of p53 gene was directly controlled by the single promoter of adenovirus vector, and the poly adenosine tall signal was added, thus an intact expression cassette was constituted.
2. Application: This recombinant medicine can many induce the abnormal hyperplastic cells expressing normal P53 protein, thus effectively repressing the cell reproduction and curing hyperplastic scars including cheloid.

The recombinant medicine of this invention, was firstly transfected the specifically genetically engineered cells. Then the cells were grown, concentrated and purified into recombinant p53 adenovirus anti-hyperplasia injection which could be used in clinical application.

The 293 cells used in this invention (ATCC CRL-1573, 32th generation, bought from ATCC, June 13^{th}, 1997) was screened from human embryonic kidney epithelial cells which was transformed by Adenovirus 5 (Ad5) DNA and contained 11% of the gene (including E1a) from Ad5 5' end. The cells were highly permissive for infection by adenovirus, and also permissive for growth of adenovirus.

The main contribution of this invention lies in taking advantage of the human tumor suppressor gene p53 which could suppress the growth of many abnormal hyperplastic cells. The suppressor gene was cloned into E1⁻ adenovirus and was locally injected into body. Adenovirus help p53 gene enter the hyperplastic tissues. The expressed p53 protein could inhibit growth of the abnormal hyperplastic cells or even kill the abnormal cells. This invention also provided a method to prepare this recombinant p53 adenovirus product, which solved the problem caused by the instability of P53 protein (half-life = 20 minutes) *in vitro*.

The recombinant p53 adenovirus carried the human tumor suppressor gene p53 and expressed it directly in the abnormal hyperplastic cells, thus solved the problem that recombinant genetically engineered product could not be made *in vitro* because instability of p53 protein. Using the adenovirus for treatment of hyperplastic diseases, P53 protein could be expressed *in vivo* continuously and highly efficiently. Furthermore, protein molecular modifications *in vivo* including phosphorylation, folding, and polymerization were equal to those in eukaryotic cells. The recombinant p53 adenovirus of this invention could be used to introduce the expression of p53 gene in eukaryotic cells by directly introducing the gene to the hyperplastic tissue to express protein, effectively using the patient as a source for producing human tumor suppressor factor P53 protein. This method successfully introduced the foreign p53 gene into the human body and allowed it to highly express in the hyperplastic tissue. This has made gene therapy of hyperplastic diseases possible.

### Detailed Description of the Figures

Figure 1 is the schematic process of the construction of the recombinant medicine
Figure 2 is the flow chart of the experimental protocols for the production of the recombinant medicine
Figure 3 is the stability testing diagram of agarose gel electrophoresis of the recombinant gene after generations of passage, which was made by PCR to amplify the recombinant p53 adenovirus using 5' CCACGACGGTGACACGCTTC and 5' CAAGCAAGGGTTCAAAGAC as primer, and p53cDNA as template.
Figure 4 is the result analysis diagram of agarose gel electrophoresis of the PCR amplification of virus DNA, which was obtained 36 hours after cell 293 was infected by recombinant gene (preserved by SiBiono Corp., preservation No.: No-1, same as the following).
Figure 5 is the Western blot analysis result 36 hours after the Hep-2 and H1299 cellswere infected by recombinant adenovirus.
Figure 6 is the primary cultured fibroblasts of human hypertrophic scar *in vitro*
Figure 7 is the characterization of the fibroblast cells using S-P staining and vacuum.
Figure 8 is the microscopic photo of the killing effect of the recombinant gene to the scar fibroblast cells *in vitro*.
Figure 9 is the electron micrograph of the killing effect of the recombinant gene to the scar fibroblast cells *in vitro*.
Figure 10 includes the pictures of the effect of recombinant gene to the cheloid patient before and after treatment.

### Detail Description of Embodiments

The following embodiments are further descriptions for this invention. The practice of this invention is not limited to these embodiments.

### Experiment 1:

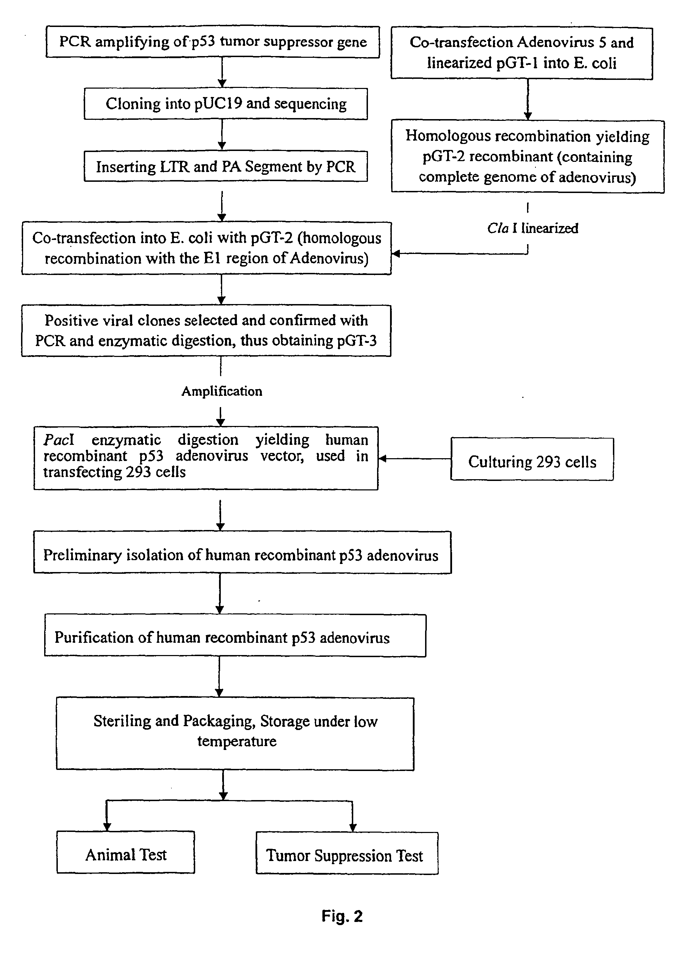

Construction and testing of the recombinant p53 adenovirus, as described in figure 1 and 2.
1. Two primers were devised according to the published full sequence of p53 cDNA:
   The two primers were 5' ATGGAGGAGCCGCAGTCAGATC and 5' ATATCTGCA GAATTCCAGCAC. Linker sequence was attached to both ends. Human p53 gene was PCR amplified using HeLa cell cDNA as a template. The experimental conditions were as follows:
   For the first cycle, the DNA was denatured for 4 minutes at 94°C, annealing for 1 minute at 58°C, and then extended for 2 minutes at 72°C. For each of the rest of the cycles, the DNA was denatured for 1 minute at 94°C, annealing for 1 minute at 58°C, and then extended for 2 minutes at 72°C. There were 30 cycles total.
   Thus a large amount of p53 gene fragments were obtained. The p53 gene was then tested using agarose gel electrophoresis. The full sequence of p53 gene was recycled from the gel, purified, cut by restriction enzyme and inserted into pUC19 vector which was cut by the same enzyme. The fragment was then sequenced.
   The base sequence of the expressing section tested the same as the predicted amino acid sequence (concurrent with GenBank Acc XM_058834). Finally the fragment was cleaved by restriction enzyme and recollected.
2. LTR and PA sequences were PCR amplified. Their primers were respectively:
   5'TCTGACATGCGATGTCGACTCG □
   5' CGGCAGTGACCCGGAAAGCAG□
   5' TCACAGAGTGCA TTGTGAGGG□
   5' GCTCTAGCGTGGGGATCCC.

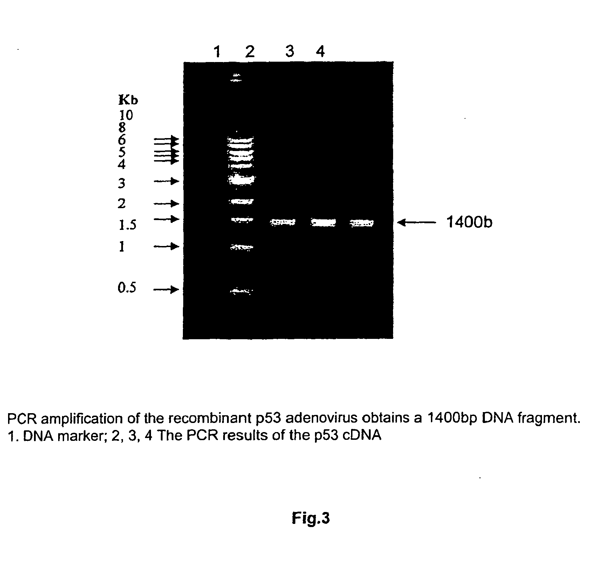

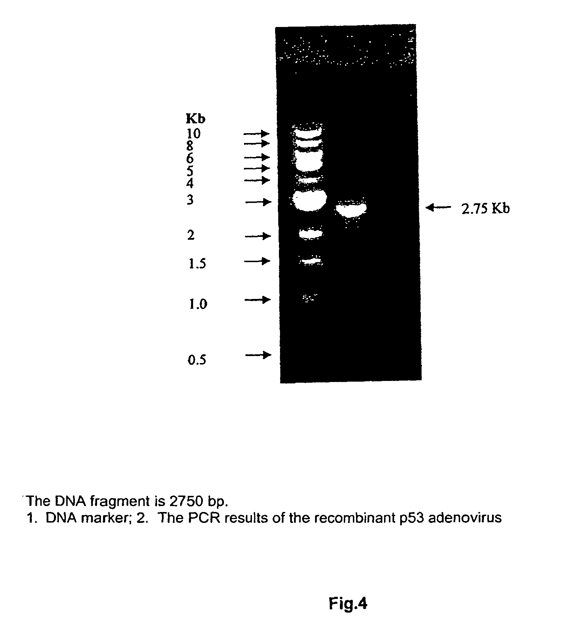

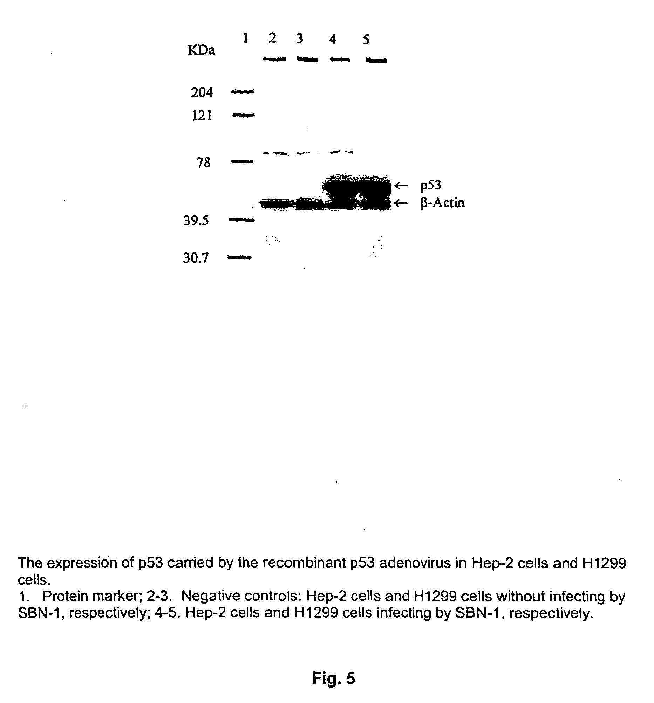

   Linker sequences were attached to 5' primer and 3' primer.
   LTR and PA were PCR amplified under the same annealing conditions described above. The amplified fragments were purified and tested by sequencing.
3. Adenovirus E1 sequence was PCR amplified separately under the same PCR conditions described above. Enzyme restriction sites for *Bam* HI and *Eco* RI were respectively linked to primers on both ends. The fragments were tested after being amplified.
4. The fragment from step 1 and the two fragments from step 2 were linked by PCR reaction respectively. The experimental conditions were as described above. PCR replication product LTR-p53-PA was obtained. The resulting sequence was tested by sequencing.
5. The fragment from step 3 and LTR-p53-PA from step 4 were linked by T4 DNA ligase. The resulting sequence was p53 gene cassette.
6. Inverted terminal repeat (IRT) sequences from both ends of adenovirus were PCR amplified under the same experimental conditions as described above. After being tested by sequencing, the resulting fragments were cloned to pUC18 vector. Thus the recombinant vector pGT-1 was obtained.
7. *E. coli* BJ5183 was co-transfected with recombinant vector pGT-1 and wild type adenovirus 5 (ATCC-VR-5, adenovirus 75, titer: 10(6.75) TCID (50)/ml) DNA. After being kept at 4°C for 1 minute, the transfected bacteria were then heat shocked at 42°C for 50 seconds, incubated at 4°C for 1 minute, then combined with 1 ml LB media and incubated for 1 hour. The engineered bacteria were then spread on agar medium containing ampicillin and incubated for 24 hours. Single cells were picked by aseptic toothpick and put into a bottle with LB media, culture for 24 hours. Plasmid was extracted by common methods and screened by *Pac*l. The positive clones were pGT-2 (containing the full sequence of Ad5).
*8. E. coli* BJ5183 was co-transfected by recombinant vector pGT-2 and p53 gene cassette. The growing condition, screening and characterization methods were described above. Positive clones were pGT-3, which contained the adenovirus full gene sequence and inserted p53 expression cassette. The vector sequence originated from pUC18 was discarded after the clones were linearized by *Pac*l*.*
9. The positive linear plasmid were purified by CsCl and then used to transfect 293 cells using CaCl₂. Cells were collected after 7 days. The cells were centrifuged at 1000rpm for 15 minutes. The supernatant was discarded. Cells were lysed 3 times at 37°C-80°C. It was again centrifuged at 4000rpm for 30 minutes. Precipitates were discarded. The supernatant was infected again to amplify the virus, and lysed the same way as described above. The resulting supernatant was density gradient centrifuged with CsCl 60000rpm at 4°C for 16 hours. The band of recombinant adenovirus was extracted by No. 7 needle. The DNA fragment was added with N1H buffer and dialyzed at 4°C for 4 hours in a Spectra MW6000 dialysis bag. The DNA solution was sterilized by passing through a 0.25µm filter. Then the DNA solution were packed and stored at -80°C. Part of the resulting product was used in plaque assay and virus titer test.
10. Structure stability test for the recombinant adenovirus. The virus genomic DNA was obtained after generations of reproduction. The DNA fragments were PCR amplified using primers from both ends of p53 which were 5'CCACGACGGTGACACGCTTC and 5' CAAGCAAGGGT TCAAAGAC. The results of agarose gel electrophoresis are shown in figure 3. Adenovirus arms at both sides of recombinant adenovirus were devised as primers: 5' TTT CTC AGG TGT TTT CCG C and 5' CAT CGT ACC TCA GCA CCT TC.. The results of PCR were shown in figure 4. The results above indicated that the structure of the recombinant p53 adenovirus was stable after many generations of reproduction.
11. The p53 gene expression test in Hep-2 and H1299 cells. 36 hours after being transfected by recombinant adenovirus, the Hep-2 and H1299 cells were lysed by common methods. The result of the western blot using P53 protein specific antibody was shown in figure 5.

### Experiment 2:

The killing effect of recombinant adenovirus to fibroblast cells:

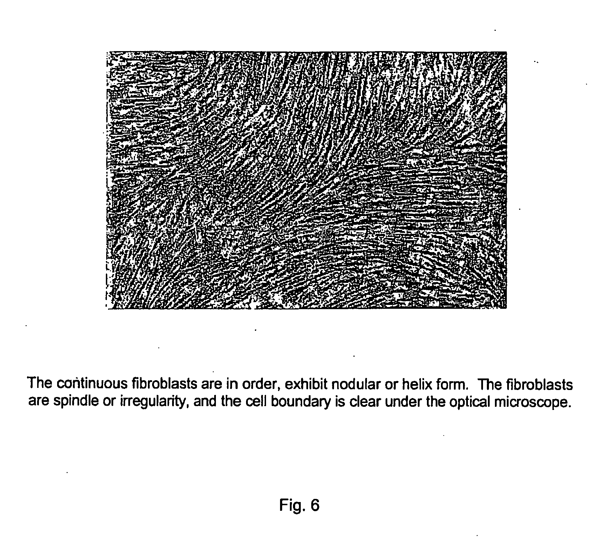

The culture of scar fibroblast cells *in vitro* (see figure 6): The Scar skin was obtained from surgery and cut into small pieces with size of 0.5-1cm³ for each in aseptic conditions. The skin pieces were then immediately put into culture solutions with 1000U/ml ampicillin and streptomycin. The tissues were washed twice with PBS (with ampicillin and streptomycin) to remove fat and connective tissues. Then the tissues were washed again several times with D-Hank solution until oil-free and clear. Next, the skin pieces were cut again until the size of 1mm³ for each piece. A few drops of serum were applied on the tissues and the pieces were spread out on the wall of the culture bottle. The bottle was then turned until the side with the tissues facing upwards and DMEM culture solution with 10% FBS was added (note: the tissues and the solution were separate). Then the bottle was sat in oven with 37°C and 5% CO₂ with the side of tissues facing upwards. 6-8hr later, the bottle was turned gently. The bottle was then sat for 3-4 days. The solution was changed every 3-4 days afterwards. New cells grew around the tissue pieces in 10 days and cell clusters came into being. The cells covered the whole bottle in about one month, forming single layer cells.

### Cell types characterization using S-P staining and vacuum method

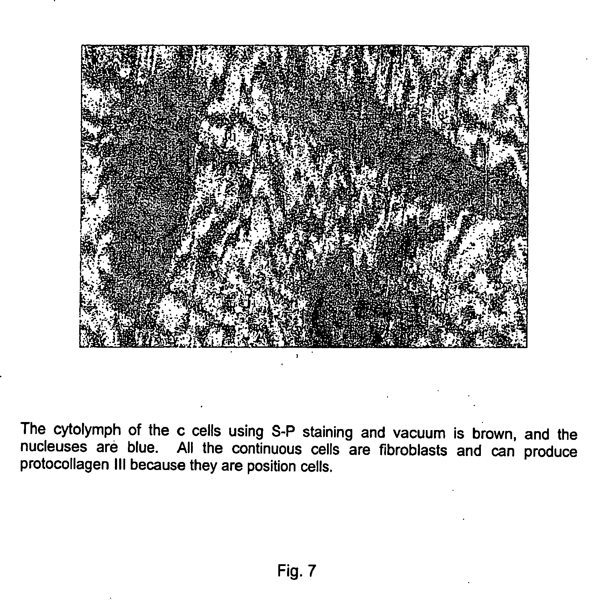

Sample preparations: The cells were digested using trypsin and made into cell suspension with concentration of 10000 cells/ml. Cover slips of 18x18mm were put in media dishes with size of 55mm. Two drops of cell suspension were put on each cover slip. And the cover slips were sat in oven with CO₂ for 6-8hrs and then washed thoroughly with PBS for 3 times. Next, the cover slips were put in pure acetone, fixed in room temperature for 15 min, and washed with PBS for 3 times. Immunochemical cell staining using S-P in vacuum (see figure 7): The cover slips were sat with the side of cells facing upwards on top of the slides. 50µl of mouse anti-human were applied on each slide. Same amount of PBS was added to the negative control group. The slides were put in vacuum oven with 66.7kPa for 10min and washed again with PBS for 3 times. Each slide was applied with 50µl of streptomycin biotin protein-peroxidase solution, sat in vacuum oven for 10min, washed with PBS for 3 times, re-stained with haematoxylin for 1min, applied with 1% alcohol for a few seconds, dehydrated with alcohol, applied with dimethylbenzene, sealed with gum and finally observed under microscope.

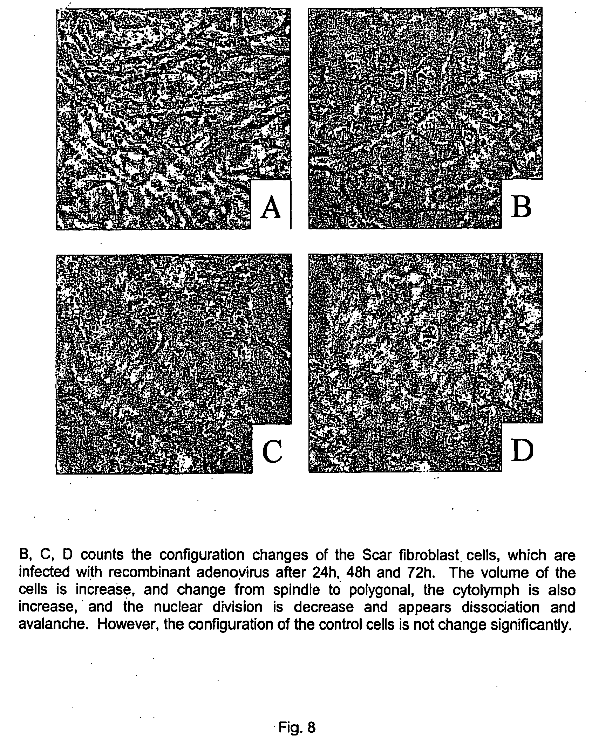

Observation of cell changes in microscope (see figure 8): 5×10⁵ counts of Scar fibroblast cells were inoculated in a 25cm² bottle, and cultured for 24 hours. Then the culture solution was changed and the cells were infected with recombinant adenovirus of 200MOI. The configuration changes of cells were observed under microscope after 24h, 48h, and 72h.

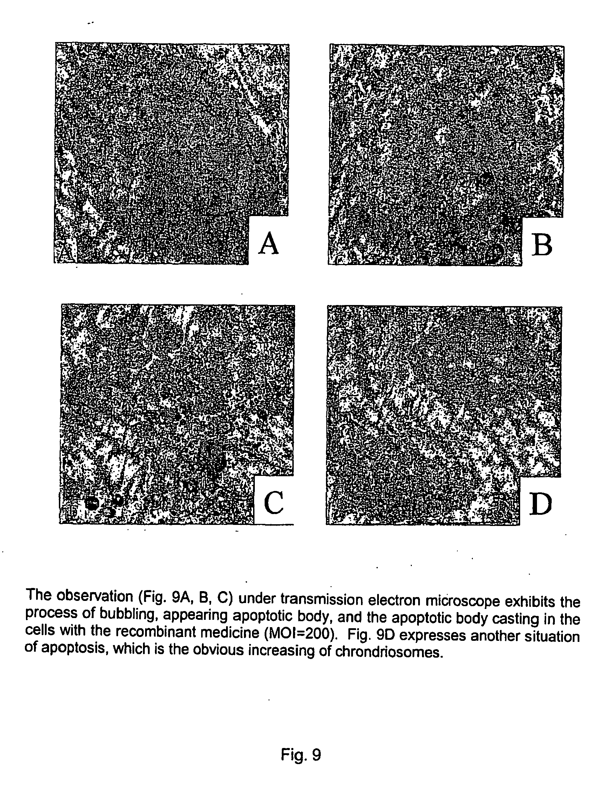

The change of cells structure in electronic microscope (see figure 9): 5×10⁵ counts of scar fibroblast cells were inoculated in a 25cm² bottle, and cultured for 24 hours. Then the culture solution was changed and the cells were infected with recombinant adenovirus of 200MOI for 48h. The cells were digested by trypsin and the cells suspension was collected in agarose tube. The tubes were then centrifuged in 2000r/min for 15min to form the cells clump. The cells were then fixed with 2% of glutaraldehyde and 1% of osmium tetroxide. The cell clumps wrapped in agar were then dehydrated, permeated and wrapped with epoxy resin, thin-sliced, stained, observed and photoed under transmission electron microscope.

### Experiment 3:

The treatment results of this recombinant adenovirus to cheloid in clinical research of scar gene therapy.

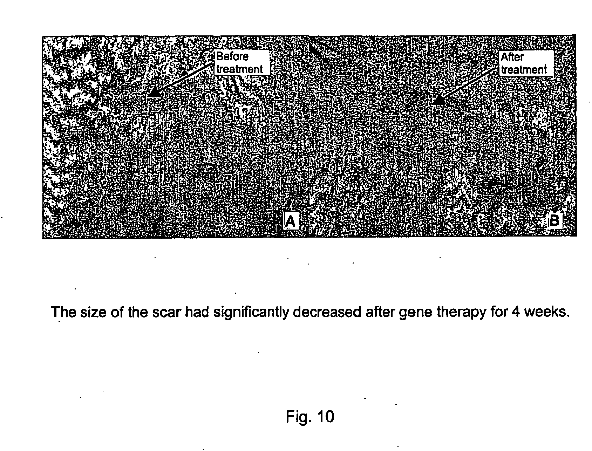

As shown in figure 10A and 10B, a female cheloid patient had scars cut off from her left chest after acnes. However, neoplastic scar was regenerated after the surgery. The local regeneration occurred in 3 years. Steroid and other common treatments showed no effect. The volume of the scar on her chest is 2×1×1cm³ (see figure 10A). The size of the scar had significantly decreased after gene therapy for 4 weeks. No other obvious side-effect showed except for self-limited fever. The recombinant adenovirus treatment for cheloid had been proved safe by clinical experiments.

## Claims

1. Use of a recombinant adenovirus vector comprising a human tumor suppressor p53 gene expression cassette for the manufacture of a medicament for treating hyperplastic scar wherein thep53 expression cassette is composed of Rous Sarcoma Virus LTR promoter-5' cis-acting sequence-p53 cDNA-3'cis acting sequence-polyadenosine .

2. The use according to claim 1, wherein the recombinant adenoviral vector comprises SEQ ID No 1 and is obtainable by a method for producing said vector in prokaryotic cells by homologous recombination, including the steps of:
1) obtaining the recombinant vector pGT -2 by homologous recombination of an adenovirus and the plasmid pGT -1 in prokaryotic cells wherein said plasmid pGT-1 contains two adenovirus inverted terminal repeats on both ends;
2) obtaining the recombinant vector pGT -3 by homologous recombination of pGT-2 and the artificial sequence "adenovirus right arm/promoter-p53cDNA-poly A/adenovirus left arm" in prokaryotic cells; and
3) obtaining the recombinant p53 adenovirus vector by discarding the prokaryotic sequence using endonuclease Pac 1

3. The use according to claim 2 wherein the prokaryotic cell is E. Coli.

4. The use according to claim 1 wherein the pathological scar is cheloid.

5. The use according to claim 1 wherein the medicament manufactured is in the form of an injection solution.

6. A recombinant adenovirus vector comprising a human tumor suppressor p53 gene expression cassette wherein thep53 expression cassette is composed of Rous Sarcoma Virus LTR promoter-5' cis-acting sequence p53 cDNA-3'cis acting sequence-polyadenosine for use treating in hyperplastic scar.

7. A recombinant adenovirus vector wherein the hyperplastic scar is for use according to claim 6 a cheloid scar.

8. A recombinant adenovirus vector for use according to claim 6 in the form of an injectable section.

9. A recombinant adenoviral vector for use according to claim 6 wherein the vector comprises SEQ ID No 1 and is obtainable by a method for producing said vector in prokaryotic cells by homologous recombination, including the steps of:
1) obtaining the recombinant vector pGT-2 by homologous recombination of an adenovirus and the plasmid pGT -1 in prokaryotic cells wherein said plasmid pGT-1 contains two adenovirus inverted terminal repeats on both ends;
2) obtaining the recombinant vector pGT -3 by homologous recombination of pGT-2 and the artificial sequence "adenovirus right arm/promoter-p53cDNA-poly A/adenovirus left arm" in prokaryotic cells; and
3) obtaining the recombinant p53 adenovirus vector by discarding the prokaryotic sequence using endonuclease Pac 1

10. A recombinant adenoviral vector for use according to claim 9 wherein the procaryotic cells are E. Coli cells.

## Patentansprüche

1. Verwendung eines rekombinanten Vektors von Adenovirus, der eine Expressionskassette für das humane Tumorsuppresor-Gen p53 aufweist, und zwar für die Herstellung eines Medikaments zur Behandlung einer hyperplastischen Narbe, wobei die Expressionskassette von p53 aufgebaut ist aus Rous Sarkom Virus LTR Promotor-5' cis-acting Sequenz p53 cDNA-3' cis-acting Sequenz Polyadenosin.

2. Verwendung nach Anspruch 1, wobei der rekombinante adenovirale Vektor SEQ ID No 1 aufweist und erhalten werden kann mit Hilfe eines Verfahren zum Erzeugen des Vektors in prokaryotischen Zellen durch homologe Rekombination, welches Verfahren die Schritte einschließt:
1) Erhalten des rekombinanten Vektors pGT-2 durch homologe Rekombination eines Adenovirus und des Plasmids pGT-1 in prokaryotischen Zellen, wobei das Plasmid pGT-1 an beiden Enden zwei Adenovirus invertierte terminale Wiederholungen enthält;
2) Erhalten des rekombinanten Vektors pGT-3 durch homologe Rekombination von pGT-2 und der artifiziellen Sequenz "Adenovirus rechter Arm / Promotor p53 cDNA-Poly A / Adenovirus linker Arm" in prokaryotischen Zellen; sowie
3) Erhalten des rekombinanten Vektors p53 von Adenovirus durch Abstoßen der prokaryotischen Sequenz unter Verwendung von Endonuclease Pac 1.

3. Verwendung nach Anspruch 2, wobei die prokaryotische Zelle *E. coli* ist.

4. Verwendung nach Anspruch 1, wobei die pathologische Narbe ein Cheloid ist.

5. Verwendung nach Anspruch 1, wobei das Medikament in Form einer Injektionslösung hergestellt wird.

6. Rekombinanter Vektor von Adenovirus, der eine Expressionskassette eines humanen Tumorsuppresor-Gens p53 aufweist, wobei die Expressionskassette von p53 aufgebaut ist aus Rous Sarkom Virus LTR Promotor-5' cis-acting Sequenz p53 cDNA-3' cis-acting Sequenz Polyadenosin, zur Verwendung bei der Behandlung von hyperplastischen Narben.

7. Rekombinanter Vektor von Adenovirus zur Verwendung nach Anspruch 6, wobei die hyperplastische Narbe eine Cheloid-Narbe ist.

8. Rekombinanter Vektor von Adenovirus zur Verwendung nach Anspruch 6 in Form einer injizierbaren Lösung.

9. Rekombinanter adenoviraler Vektor zur Verwendung nach Anspruch 6, wobei der Vektor SEQ ID No 1 aufweist und erhalten werden kann mit Hilfe eines Verfahrens zum Erzeugen des Vektors in prokaryotischen Zellen durch homologe Rekombination, welches Verfahren die Schritte einschließt:
1) Erhalten des rekombinanten Vektors pGT-2 durch homologe Rekombination eines Adenovirus und des Plasmids pGT-1 in prokaryotischen Zellen, wobei das Plasmid pGT-1 an beiden Enden zwei Adenovirus invertierte terminale Wiederholungen enthält;
2) Erhalten des rekombinanten Vektors pGT-3 durch homologe Rekombination von pGT-2 und der artifiziellen Sequenz "Adenovirus rechter Arm / Promotor p53 cDNA-Poly A / Adenovirus linker Arm" in prokaryotischen Zellen; sowie
3) Erhalten des rekombinanten Vektors p53 von Adenovirus durch Abstoßen der prokaryotischen Sequenz unter Verwendung von Endonuclease Pac 1.

10. Rekombinanter Vektor von Adenovirus zur Verwendung nach Anspruch 9, wobei die prokaryotischen Zellen von *E. coli* sind.

## Revendications

1. Utilisation d'un vecteur d'adénovirus recombiné comprenant une cassette d'expression du gène p53 suppresseur tumoral humain pour la fabrication d'un médicament pour traiter une cicatrice hyperplasique, dans laquelle la cassette d'expression p53 est composée de promoteur LTR du virus du sarcome de Rous-séquence agissant en cis 5'-ADNc p53-séquence agissant en cis 3'-polyadénosine.

2. Utilisation selon la revendication 1, dans laquelle le vecteur adénoviral recombiné comprend SEQ ID N° 1 et peut être obtenu par un procédé de production dudit vecteur dans des cellules procaryotes par recombinaison homologue, incluant les étapes consistant à :
1) obtenir le vecteur recombiné pGT-2 par recombinaison homologue d'un adénovirus et du plasmide pGT-1 dans des cellules procaryotes, dans laquelle ledit plasmide pGT-1 contient deux répétitions terminales inversées d'adénovirus aux deux extrémités ;
2) obtenir le vecteur recombiné pGT-3 par recombinaison homologue de pGT-2 et de la séquence artificielle « bras droit d'adénovirus/promoteur-ADNc p53-polyA/bras gauche d'adénovirus » dans des cellules procaryotes ; et
3) obtenir le vecteur d'adénovirus p53 recombiné en supprimant la séquence procaryote en utilisant l'endonucléase Pac I.

3. Utilisation selon la revendication 2, dans laquelle la cellule procaryote est E. Coli.

4. Utilisation selon la revendication 1, dans laquelle la cicatrice pathologique est chéloïdienne.

5. Utilisation selon la revendication 1, dans laquelle le médicament fabriqué est sous la forme d'une solution d'injection.

6. Vecteur d'adénovirus recombiné comprenant une cassette d'expression du gène p53 suppresseur tumoral humain, dans lequel la cassette d'expression p53 est composée de promoteur LTR du virus du sarcome de Rous-séquence agissant en cis 5'-ADNc p53-séquence agissant en cis 3'-polyadénosine destiné à être utilisé dans le traitement d'une cicatrice hyperplasique.

7. Vecteur d'adénovirus recombiné destiné à être utilisé selon la revendication 6, dans lequel la cicatrice hyperplasique est une cicatrice chéloïdienne.

8. Vecteur d'adénovirus recombiné destiné à être utilisé selon la revendication 6 sous la forme d'une solution injectable.

9. Vecteur adénoviral recombiné destiné à être utilisé selon la revendication 6, dans lequel le vecteur comprend SEQ ID N° 1 et peut être obtenu par un procédé de production dudit vecteur dans des cellules procaryotes par recombinaison homologue, incluant les étapes consistant à :
1) obtenir le vecteur recombiné pGT-2 par recombinaison homologue d'un adénovirus et du plasmide pGT-1 dans des cellules procaryotes, dans lequel ledit plasmide pGT-1 contient deux répétitions terminales inversées d'adénovirus aux deux extrémités ;
2) obtenir le vecteur recombiné pGT-3 par recombinaison homologue de pGT-2 et de la séquence artificielle « bras droit d'adénovirus/promoteur-ADNc p53-polyA/bras gauche d'adénovirus » dans des cellules procaryotes ; et
3) obtenir le vecteur d'adénovirus p53 recombiné en supprimant la séquence procaryote en utilisant l'endonucléase Pac I.

10. Vecteur adénoviral recombiné destiné à être utilisé selon la revendication 9, dans lequel les cellules procaryotes sont des cellules de E. Coli.
